# EUROPEAN PATENT APPLICATION

(11) **EP 4 163 028 A1**
(43) Date of publication of application: **12.04.2023**
(21) Application number: 20939466.7
(22) Date of filing: 05.06.2020
(51) Int. Cl.: B21C 37/04, C22C 1/02, C22C 23/04, C22C 23/00, C22F 1/06

(54) **METHOD FOR PREPARING BIOMEDICAL MAGNESIUM ALLOY WIRE MATERIAL**

(71) Applicant: Institute of Metal Research, Chinese Academy of Sciences, Shenyang, Liaoning 110016 (CN); Sichuan Megall Medical Devices Co., Ltd, Chengdu, Sichuan 610200 (CN)
(72) Inventor: MA, Zheng, Chengdu, Sichuan 610200 (CN); TAN, Lili, Chengdu, Sichuan 610200 (CN); YANG, Ke, Chengdu, Sichuan 610200 (CN)
(74) Representative: Ipey
(86) International application number: PCT/CN2020/094573
(87) International publication number: WO 2021/243683

(57) **Abstract**

The disclosure relates to the technical field of preparing the metal material of magnesium alloy, and particularly provides a method for preparing biomedical magnesium alloy wires. The magnesium, zinc, and neodymium alloys are subject to smelting, casting, rolling, and other processes to prepare plates. The plates are subjected to a special mechanical stirring process to prepare a processing zone with the same thickness as the plates. After machining, the processing zone is used as the final product of the wire or drawn in multiple passes to finally form the wire with the required diameter. By introducing rolling and mechanical stirring processes, the disclosure improves the forming property of the wire, so that the alloy grains are significantly refined, the size of the second phase is greatly reduced and most of them are solid-soluble in the matrix, the strength of the obtained wire, and especially the elongation, is greatly improved, and better corrosion resistance is obtained, which meet the performance requirements of medical magnesium alloy wire.

## Description

### TECHNICAL FIELD

The disclosure relates to the technical field of preparing the metal material of magnesium alloy, and particularly provides a method for preparing biomedical magnesium alloy wires.

### BACKGROUND

As a degradable material with good biosafety, magnesium alloy wire can be gradually degraded and metabolized in vivo until it disappears after the implantation function is realized, avoiding the second removal surgery. It has wide application prospects in the field of biomedicine and can be used as implantation materials for sternal connection in cardiac surgery, cartilage connection in plastic surgery, gastrointestinal anastomosis in general surgery, tracheal stent support, esophageal stent support, and duodenal food filtration.

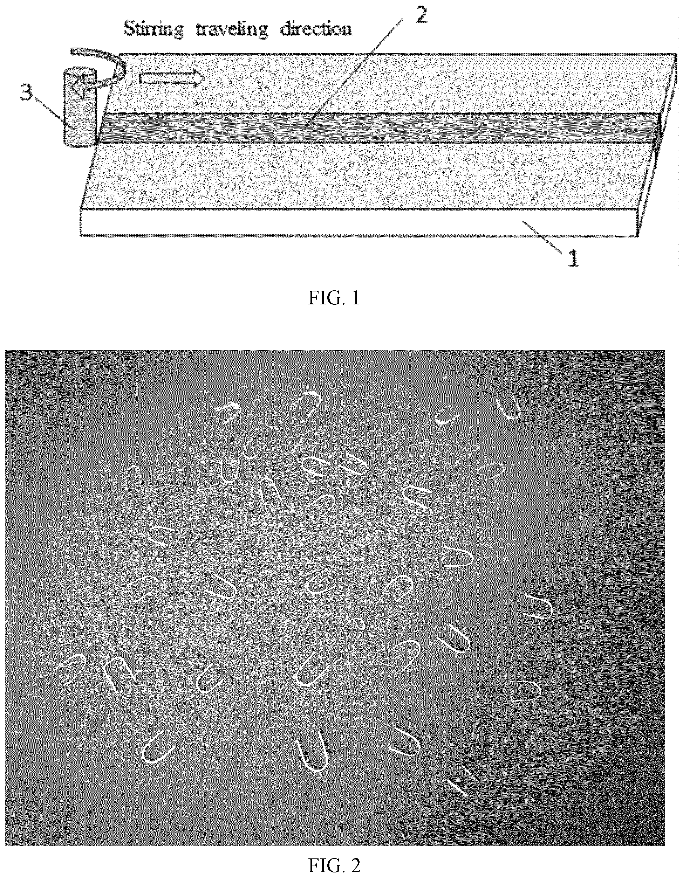

The strength, plasticity, and corrosion rate of magnesium alloy wire are the key to its medical application. The microstructure, mechanical properties, and corrosion resistance of fine magnesium alloy wire are not improved by drawing. In addition, the different additive elements in magnesium alloy wire affect its wire forming property. China Patent Application No. 201810068159.5 discloses a method for preparing Mg-Zn-Mn-X (X=Ag, Sr, Ca, Bi) alloy wires by crushing and drawing with a large extrusion ratio, and the obtained wires have small diameter, uniform microstructure, and good mechanical properties. China Patent Application No. 201710159114.4 discloses a method for preparing equal-diameter angular extrusion combined with multi-pass drawing, which obtains a magnesium-zinc alloy wire with mechanical and corrosion properties superior to those obtained by drawing alone. However, the size of magnesium wire prepared by equal-diameter angular extrusion process is limited, and the material processing loss is large, making it not suitable for large-scale production. Based on the conventional drawing for magnesium alloy wires, the disclosure introduces a rolling process instead of the drawing process during the primary processing of large-size materials, changes the stress of the material in plastic deformation, and improves the forming property and processing efficiency of magnesium alloy to a certain extent. When the magnesium alloy is rolled into a thin plate, a mechanical stirring process is introduced. The principle is that a stirring needle with a threaded cylinder mechanically stirs from the center of one end to the center of the other end of the plate, and the relative motion between the stirring needle and the material is used to cause strong plastic deformation of the material, so as to improve the microstructure and performance of the material. The schematic diagram is shown in Figure 1. The zone subjected to the mechanical stirring process can be used as the final material of the wire or further drawn.

### SUMMARY

The purpose of the disclosure is to provide a method for preparing magnesium alloy wires for medical use given the difficulty in preparing and forming magnesium alloy wire, and in meeting the requirements of medical wire in final use performance such as strength, plasticity, and corrosion resistance.

Technical solution of the disclosure:
A method for preparing biomedical magnesium alloy wires changes the traditional wire drawing process, and combines the three-in-one process of rolling, mechanical stirring, and drawing to realize the optimization of magnesium alloy wire preparation and the improvement of overall service performance, including the following operation steps:
(1) Smelt pure magnesium and other alloying elements into liquid metals in proportion, stir evenly and remove slags;
(2) Cast the alloy solution in step (1) into flat ingots, and remove surface defects and impurities;
(3) Put the flat ingots in step (2) under homogenizing heat treatment at 250-400 °C for 2-5h;
(4) Process the flat ingots in step (3) into magnesium alloy plates with a thickness of 70-100 mm, a width of 540-730 mm, and a length of 400-1,200 mm by hot rolling, in which the tapping temperature of the metal is 470-510 °C and the heating time is 3-6 h;
(5) Process the magnesium alloy plates in step (4) into magnesium alloy plates with a thickness of 10-20 mm, a width of 540-730 mm, and a length of 400-1,200 mm by hot rolling, in which the tapping temperature of the metal is 440-470 °C and the heating time is 2-5 h;
(6) Process the magnesium alloy plates in step (5) into magnesium alloy plates with a thickness of 2-8 mm, a width of 540-730 mm, and a length of 400-1,200 mm by hot rolling, in which the tapping temperature of the metal is 380-440 °C and the heating time is 2-4 h;
(7) Process the magnesium alloy plates in step (6) by a mechanical stirring process to prepare a stirring plastic deformation zone of the same thickness as the magnesium alloy plates;
(8) Cut the stirring plastic deformation zone in the magnesium alloy plates in step (7) and machine it into a bar with a diameter of Φ2-8 mm;
(9) Draw the bar in step (8) into a wire in multiple passes, accompanying annealing heat treatment with the temperature of 280-320 °C, the time of 10-60 min, the single-pass deformation of 15-25%, and the drawing speed is 0.01-0.05 m/s.

According to the method for preparing biomedical magnesium alloy wires, in step (1), other alloying elements include Zn: 0.2%-2.5% and Nd: 0.2%-2.5% by weight.

According to the method for preparing biomedical magnesium alloy wires, in the mechanical stirring process in step (7), the direction of mechanical stirring is along the rolling direction of the plates at the rotating speed of 400-1,200 rpm, a stirring needle with a diameter of 1-5 mm is arranged in the bottom center of the concave shaft shoulder with a diameter of 15-25 mm and travels at a speed of 80-120 mm/min, the inclination angle between the axis of the stirring needle and the normal line of the surface of the magnesium alloy plate workpiece is 2.6-3°, and the pressing amount during stirring is kept at 0.1-0.2 mm.

According to the method for preparing biomedical magnesium alloy wires, in step (7), the stirring plastic deformation zone of the mechanical stirring process is machined to prepare the wire as a final product, or as an intermediate product to be further drawn and processed into the wire in multiple passes.

The design idea of the disclosure is:
Given the poor wire forming property of magnesium alloy at present, the final product is difficult to achieve the mechanical properties and corrosion resistance of medical materials. The disclosure changes the traditional drawing process, and combines the rolling and mechanical stirring processes. Firstly, reduce the forming difficulty of magnesium alloy by rolling to ensure the large-scale production of materials, and make it possible for the subsequent mechanical stirring process; Then through a mechanical stirring process, further improve the microstructure of the alloy, and increase the strength, plasticity, corrosion resistance and subsequent drawing property of the alloy; Finally, obtain the final target wire by several drawing processes. The above jointly ensure that the wire has good plastic forming property, as well as mechanical strength and corrosion resistance for medical use.

The disclosure also provides a method for preparing biomedical magnesium alloy wires, comprising the following contents:
A. Mechanically stir the magnesium alloy plates to prepare a stirring plastic deformation zone with the same thickness as the magnesium alloy plates;
B. Prepare the stirring plastic deformation zone into a bar with a diameter of Φ2-8 mm;
C. Draw and process the bar into a wire;
wherein, in the mechanical stirring process, the direction of mechanical stirring is along the rolling direction of the plates at the rotating speed of 400-1,200 rpm, a stirring needle with a diameter of 1-5 mm is arranged in the bottom center of the concave shaft shoulder with a diameter of 15-25 mm and travels at a speed of 80-120 mm/min, the inclination angle between the axis of the stirring needle and the normal line of the surface of the magnesium alloy plate workpiece is 2.6-3°, and the pressing amount during stirring is kept at 0.1-0.2 mm.

The disclosure also provides a method for preparing magnesium alloy plates, comprising the following operation steps:
(1) Smelt pure magnesium and other alloying elements into liquid metals in proportion, stir evenly and remove slags;
(2) Cast the alloy solution in step (1) into flat ingots, and remove surface defects and impurities;
(3) Put the flat ingots in step (2) under homogenizing heat treatment at 250-400 °C for 2-5h;
(4) Process the flat ingots in step (3) into magnesium alloy plates with a thickness of 70-100 mm, a width of 540-730 mm, and a length of 400-1,200 mm by hot rolling, in which the tapping temperature of the metal is 470-510 °C and the heating time is 3-6 h;
(5) Process the magnesium alloy plates in step (4) into magnesium alloy plates with a thickness of 10-20 mm, a width of 540-730 mm, and a length of 400-1,200 mm by hot rolling, in which the tapping temperature of the metal is 440-470 °C and the heating time is 2-5 h;
(6) Process the magnesium alloy plates in step (5) into magnesium alloy plates with a thickness of 2-8 mm, a width of 540-730 mm, and a length of 400-1,200 mm by hot rolling, in which the tapping temperature of the metal is 380-440 °C and the heating time is 2-4 h. The disclosure further provides the magnesium alloy wires prepared by the above method.

The disclosure further provides the magnesium alloy plates prepared by the above method.

The disclosure further provides an in-vivo implant, the raw material of which comprises the magnesium alloy plates above, or comprises materials prepared directly or indirectly from the magnesium alloy plates above.

The disclosure further provides an in-vivo implant, the raw material of which comprises the magnesium alloy wires above, or comprises materials prepared directly or indirectly from the magnesium alloy wires above.

Wherein the in-vivo implant comprises an anastomotic nail, a surgical suture, a cosmetic suture, a skin nail, a nerve connecting wire, a non-vascular stent, a peripheral vascular stent, a mesh, a vascular stapler, a bone screw, a bone plate, and a vascular clamp. Wherein, the bone plate and the vascular clamp are mainly made of magnesium alloy plates.

In addition, the raw material of the in-vivo implant may also comprise coating materials.

The coating material may be used to regulate the rate of degradation of the wires in vivo. The coating materials include, but are not limited to, a magnesium phosphate coating, a magnesium oxide coating, a magnesium carbonate coating, or a degradable polymer coating.

The advantages and beneficial effects of the disclosure are:
In combination with rolling, mechanical stirring, and drawing processes, the disclosure can prepare magnesium alloy wires on a large scale and in a refined manner, and finally obtain ultrafine equiaxed crystals. The quantity and size of the second phase of the material are greatly reduced and most of them are solid-soluble in the magnesium matrix, and the strength, plasticity, and corrosion resistance of the wires are greatly improved. Thus, by increasing the plasticity, the subsequent drawing process becomes easy to perform even at room temperature. The good strength, plasticity, and corrosion resistance of magnesium alloy wires can meet the requirements of clinical use and broaden the clinical application range of magnesium alloy wires.

### BRIEF DESCRIPTION OF THE DRAWINGS

FIG. 1 is a schematic diagram of the mechanical stirring process. In which, 1 represents magnesium alloy plate, 2 represents stirring plastic deformation zone, and 3 represents stirring needle.
FIG. 2 shows the morphology of the anastomotic nail prepared from the magnesium alloy wire.
FIG. 3 shows the metallographic structure of the magnesium alloy wire.
FIG. 4 shows the TEM morphology of the magnesium alloy wire.
FIG. 5 is an end face diagram of the stirring needle structure with a concave shaft shoulder. In which, (a) the bottom end face of the concave shaft shoulder is stepped, (b) the bottom end face of the concave shaft shoulder is spiral; 3 represents the stirring needle and 4 represents the concave shaft shoulder.

### DETAILED DESCRIPTION OF PREFERRED EMBODIMENT

In the specific implementation process, the disclosure prepares plates from magnesium, zinc, and neodymium alloys by smelting, casting, rolling, and other processes. The plates are subjected to a special mechanical stirring process to prepare a processing zone with the same thickness as the plates. After machining, the processing zone is used as the final product of the wire or drawn in multiple passes to finally form the wire with the required diameter. Given the difficulties in the preparation and forming of magnesium alloy wires, the insufficient final use performance such as strength, plasticity, and corrosion resistance is difficult to meet the requirements of medical wires. The forming property of magnesium alloy wires can be improved by changing the traditional drawing preparation process, introducing rolling and mechanical stirring processes, and combining them with subsequent drawing in the preparation process of magnesium alloy wires. Finally, we can obtain fine wires with a minimum diameter of 0.1 mm. In addition, through the severe plastic deformation caused by mechanical stirring, the fine equiaxed grains of the material are obtained, thus introducing the dislocation strengthening material. The solid solution of the second phase improves the plasticity of the material, as well as the corrosion resistance of the alloy.

The embodiments of the disclosure will be further described in detail as follows with reference to the drawings. The embodiments are implemented on the premise of the technical solution of the disclosure, and detailed embodiments and specific operation procedures are given, but the protection scope of the disclosure is not limited to the following embodiments.

### Embodiment 1

In the embodiment, the Mg-2Zn-0.5Nd alloy includes 2% Zn and 0.5% Nd by weight, and the rest is Mg.

Preparation method: Smelt pure magnesium, 2% Zn and 0.5% Nd by weight into liquid metal, cast it into flat ingots and remove surface defects and impurities, put the flat ingots under homogenizing heat treatment at 300 °C for 5h, and process them into magnesium alloy plates with a thickness of 70 mm, a width of 540 mm, and a length of 400 mm by hot rolling (tapping temperature of 480 °C, heating time of 4h); Then, process the plates into magnesium alloy plates with a thickness of 10 mm, a width of 540 mm, and a length of 400 mm was processed by hot rolling (tapping temperature of 440°C, heating time of 4 h); and further process the plates into magnesium alloy plates with a thickness of 2 mm, a width of 540 mm, and a length of 400 mm by hot rolling (tapping temperature of 440 °C, heating time of 2h).

As shown in FIG. 1, the magnesium alloy plate 1 is processed by a mechanical stirring process, the direction of which is along the rolling direction of the plate at the rotating speed of 800 rpm. A stirring needle 3 with a diameter of 2 mm is arranged in the bottom center of the concave shaft shoulder with a diameter of 20 mm and travels at a speed of 100 mm/min along the horizontal stirring traveling direction. The inclination angle between the axis of stirring needle 3 and the normal line of the surface of magnesium alloy plate workpiece 1 is about 2.8° (the function of this inclination angle is to facilitate the stirring needle to extend into the material for friction and stirring). The inclination direction of stirring needle 3 is opposite to the stirring traveling direction, and the pressing amount during stirring is kept at 0.15 mm. Thus, a strip zone with strong plastic deformation (stirring plastic deformation zone 2) is processed. Cut off the zone, machine it into a bar with a diameter of Φ2 mm, and draw the bar accompanying annealing heat treatment at a temperature of 280 °C for 20 min. The single-pass deformation is about 20%, and the drawing speed is 0.05 m/s. Finally, a wire with a diameter of 0.3 mm is formed.

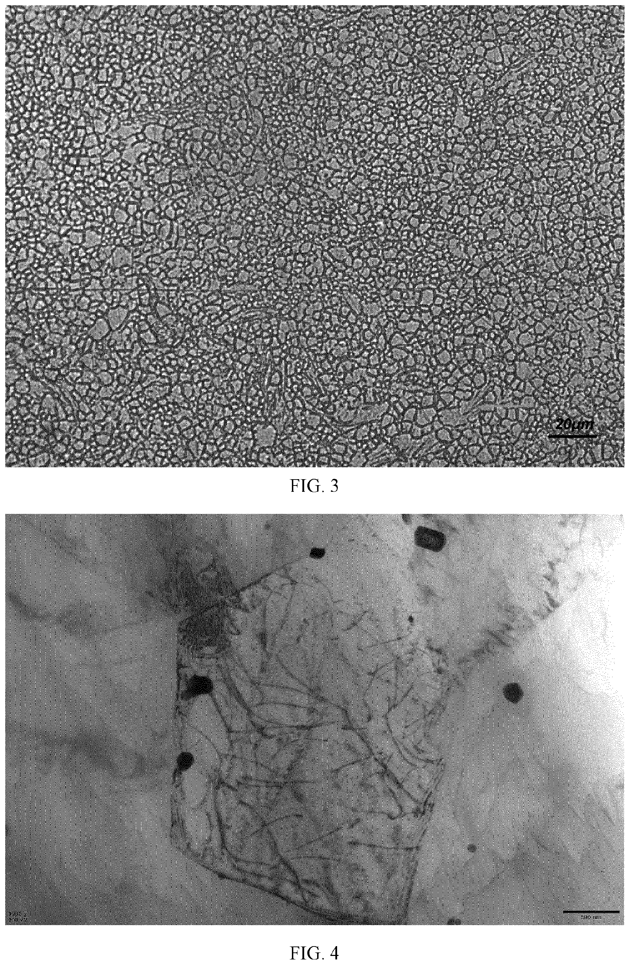

The U-shaped anastomotic nail prepared from the wire is shown in Fig. 2. The microstructure of the wire is shown in Fig. 3. The grains of the wire are equiaxed, and according to transmission electron microscope morphology of the wire in Fig. 4, the grain size of the alloy is small, ranging from 500 nm to 1 µm. Dislocations are formed inside the grains, which further strengthens the alloy and produces a very small size of the second phase, only 50-100 nm.

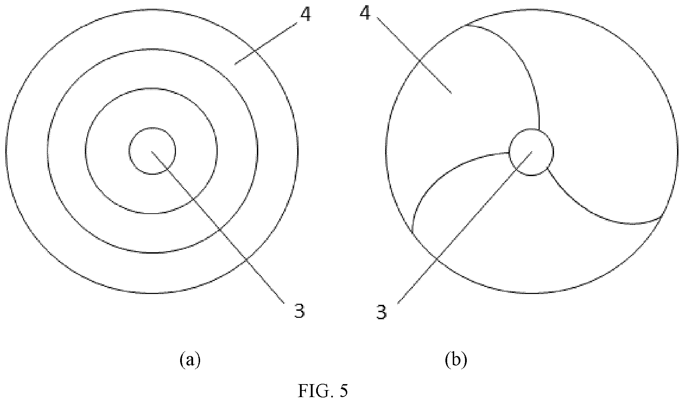

As shown in FIG. 5, the stirring needle 3 is mounted in the center of the bottom end face of the lower concave shaft shoulder 4, and the junction of the lower concave shaft shoulder 4 and the stirring needle 3 is concave to increase the contact area between the lower concave shaft shoulder 4 and the plate surface, so that the material softened below the end of the lower concave shaft shoulder 4 can be subjected to an inward force, thereby closely coupling the lower concave shaft shoulder 4 and the plasticized material.

Tensile properties (GB/T 228-2002): tensile strength is 320 MPa, and elongation is 15%.

Degradation rate (soak in Hank's solution for 30 days at 37°C): 0.33 mm/year.

### Embodiment 2

In the embodiment, the Mg-0.2Zn-2.0Nd alloy includes 0.2% Zn and 2.0% Nd by weight, and the rest is Mg.

Preparation method: Smelt pure magnesium, 0.2% Zn and 2.0% Nd by weight into liquid metal, cast it into flat ingots and remove surface defects and impurities, put the flat ingots under homogenizing heat treatment at 320 °C for 5h, and process them into magnesium alloy plates with a thickness of 70 mm, a width of 540 mm, and a length of 400 mm by hot rolling (tapping temperature of 480 °C, heating time of 4h); Then, process the plates into magnesium alloy plates with a thickness of 10 mm, a width of 540 mm, and a length of 400 mm was processed by hot rolling (tapping temperature of 440°C, heating time of 4 h); and further process the plates into magnesium alloy plates with a thickness of 2 mm, a width of 540 mm, and a length of 400 mm by hot rolling (tapping temperature of 440 °C, heating time of 2h).

As shown in FIG. 1, the magnesium alloy plate 1 is processed by a mechanical stirring process, the direction of which is along the rolling direction of the plate at the rotating speed of 800 rpm. A stirring needle 3 with a diameter of 2 mm is arranged in the bottom center of the concave shaft shoulder (Fig. 5) with a diameter of 20 mm and travels at a speed of 100 mm/min along the horizontal stirring traveling direction. The inclination angle between the axis of stirring needle 3 and the normal line of the surface of magnesium alloy plate workpiece 1 is about 2.8°. The inclination direction of stirring needle 3 is opposite to the stirring traveling direction, and the pressing amount during stirring is kept at 0.15 mm. Thus, a strip zone with strong plastic deformation (stirring plastic deformation zone 2) is processed. Cut off the zone, machine it into a bar with a diameter of Φ2 mm, and draw the bar accompanying annealing heat treatment at a temperature of 280 °C for 20 min. The single-pass deformation is about 20%, and the drawing speed is 0.05 m/s. Finally, a wire with a diameter of 0.6 mm is formed.

The grains of the wire are equiaxed, and the grain size of the alloy is small, ranging from 500 nm to 1 µm. Dislocations are formed inside the grains, which further strengthens the alloy and produces a very small size of the second phase, only 50-100 nm.

Tensile properties (GB/T 228-2002): tensile strength is 360 MPa, and elongation is 21%.

Degradation rate (soak in Hank's solution for 30 days at 37°C): 0.36 mm/year.

The embodiment results show that, by introducing rolling and mechanical stirring processes, the disclosure improves the forming property of the wire, so that the alloy grains are significantly refined, the size of the second phase is greatly reduced and most of them are solid-soluble in the matrix, the strength of the obtained wire, and especially the elongation, is greatly improved, and better corrosion resistance is obtained, which meet the performance requirements of medical magnesium alloy wire.

## Claims

1. A method for preparing biomedical magnesium alloy wires, comprising the following operation steps:
(1) Smelt pure magnesium and other alloying elements into liquid metals in proportion, stir evenly and remove slags;
(2) Cast the alloy solution in step (1) into flat ingots, and remove surface defects and impurities;
(3) Put the flat ingots in step (2) under homogenizing heat treatment at 250-400 °C for 2-5h;
(4) Process the flat ingots in step (3) into magnesium alloy plates with a thickness of 70-100 mm, a width of 540-730 mm, and a length of 400-1,200 mm by hot rolling, in which the tapping temperature of the metal is 470-510 °C and the heating time is 3-6 h;
(5) Process the magnesium alloy plates in step (4) into magnesium alloy plates with a thickness of 10-20 mm, a width of 540-730 mm, and a length of 400-1,200 mm by hot rolling, in which the tapping temperature of the metal is 440-470 °C and the heating time is 2-5 h;
(6) Process the magnesium alloy plates in step (5) into magnesium alloy plates with a thickness of 2-8 mm, a width of 540-730 mm, and a length of 400-1,200 mm by hot rolling, in which the tapping temperature of the metal is 380-440 °C and the heating time is 2-4 h;
(7) Process the magnesium alloy plates in step (6) by a mechanical stirring process to prepare a stirring plastic deformation zone of the same thickness as the magnesium alloy plates;
(8) Cut the stirring plastic deformation zone in the magnesium alloy plates in step (7) and machine it into a bar with a diameter of Φ2-8 mm;
(9) Draw the bar in step (8) into a wire in multiple passes, accompanying annealing heat treatment with the temperature of 280-320 °C, the time of 10-60 min, the single-pass deformation of 15-25%, and the drawing speed is 0.01-0.05 m/s.

2. The method for preparing biomedical magnesium alloy wires of claim 1, wherein in step (1), other alloying elements include Zn: 0.2%-2.5% and Nd: 0.2%-2.5% by weight.

3. The method for preparing biomedical magnesium alloy wires of claim 1, wherein in step (7), in the mechanical stirring process, the direction of mechanical stirring is along the rolling direction of the plates at the rotating speed of 400-1,200 rpm, a stirring needle with a diameter of 1-5 mm is arranged in the bottom center of the concave shaft shoulder with a diameter of 15-25 mm and travels at a speed of 80-120 mm/min, the inclination angle between the axis of the stirring needle and the normal line of the surface of the magnesium alloy plate workpiece is 2.6-3°, and the pressing amount during stirring is kept at 0.1-0.2 mm.

4. The method for preparing biomedical magnesium alloy wires of claim 1, wherein in step (7), the stirring plastic deformation zone of the mechanical stirring process is machined to prepare the wire as a final product, or as an intermediate product to be further drawn and processed into the wire in multiple passes.

5. A method for preparing biomedical magnesium alloy wires, comprising the following contents:
A. Mechanically stir the magnesium alloy plates to prepare a stirring plastic deformation zone with the same thickness as the magnesium alloy plates;
B. Prepare the stirring plastic deformation zone into a bar with a diameter of Φ2-8 mm;
C. Draw and process the bar into a wire;
wherein, in the mechanical stirring process, the direction of mechanical stirring is along the rolling direction of the plates at the rotating speed of 400-1,200 rpm, a stirring needle with a diameter of 1-5 mm is arranged in the bottom center of the concave shaft shoulder with a diameter of 15-25 mm and travels at a speed of 80-120 mm/min, the inclination angle between the axis of the stirring needle and the normal line of the surface of the magnesium alloy plate workpiece is 2.6-3°, and the pressing amount during stirring is kept at 0.1-0.2 mm.

6. A method for preparing magnesium alloy plates, comprising the following operation steps:
(1) Smelt pure magnesium and other alloying elements into liquid metals in proportion, stir evenly and remove slags;
(2) Cast the alloy solution in step (1) into flat ingots, and remove surface defects and impurities;
(3) Put the flat ingots in step (2) under homogenizing heat treatment at 250-400 °C for 2-5h;
(4) Process the flat ingots in step (3) into magnesium alloy plates with a thickness of 70-100 mm, a width of 540-730 mm, and a length of 400-1,200 mm by hot rolling, in which the tapping temperature of the metal is 470-510 °C and the heating time is 3-6 h;
(5) Process the magnesium alloy plates in step (4) into magnesium alloy plates with a thickness of 10-20 mm, a width of 540-730 mm, and a length of 400-1,200 mm by hot rolling, in which the tapping temperature of the metal is 440-470 °C and the heating time is 2-5 h;
(6) Process the magnesium alloy plates in step (5) into magnesium alloy plates with a thickness of 2-8 mm, a width of 540-730 mm, and a length of 400-1,200 mm by hot rolling, in which the tapping temperature of the metal is 380-440 °C and the heating time is 2-4 h.

7. Magnesium alloy wires prepared by the method of any of claims 1-5.

8. Magnesium alloy plates prepared by the method of claim 6.

9. An in-vivo implant, wherein the raw material comprises the magnesium alloy plates of claim 8 or comprises materials prepared from the magnesium alloy plates of claim 8.

10. An in-vivo implant, wherein the raw material comprises the magnesium alloy wire of claim 7, or comprises a material prepared from the magnesium alloy wire of claim 7.

11. The in-vivo implant of claim 9 or 10, wherein the in-vivo implant comprises an anastomotic nail, a surgical suture, a cosmetic suture, a skin nail, a nerve connecting wire, a non-vascular stent, a peripheral vascular stent, a mesh, a vascular stapler, a bone screw, a bone plate, and a vascular clamp.

12. The in-vivo implant of any of claims 9-11, wherein the raw material further comprises coating materials.

13. The in-vivo implant of claim 12, wherein the coating materials include a magnesium phosphate coating, a magnesium oxide coating, a magnesium carbonate coating, or a degradable polymer coating.
